# EUROPEAN PATENT APPLICATION

(11) **EP 3 315 488 A1**
(43) Date of publication of application: **02.05.2018**
(21) Application number: 17183863.4
(22) Date of filing: 28.07.2017
(51) Int. Cl.: C07C 257/12, C07C 229/10

(54) **AMINO ACID BASED AMPHOTERIC SURFACTANT**

(30) Priority: 28.10.2016 US 201662414378 P
(71) Applicant: Elé Corporation, McCook, IL 60525 (US)
(72) Inventor: BERG, Kenneth, Saint John, IN Indiana 46373 (US); OTTERSON, Richard J, Frankfort, IL Illinois 60423 (US)
(74) Representative: Barker Brettell LLP

(57) **Abstract**

Disclosed is a novel amino acid derived surfactant which is prepared by forming the salt of an alkylaminopropionic acid and a basic amino acid such as arginine which is useful in cosmetic and personal care compositions.

## Description

### PRIORITY

This application claims priority from United States Provisional Patent Application No. 62/414,378, filed 28 October 2016, the disclosure of which is incorporated herein.

### BACKGROUND OF THE INVENTION

The present invention relates to an amino acid derived surfactant. More specifically, the present invention relates to a novel amphoteric surfactant which is less irritating to the skin compared to prior art surfactants, which exhibits excellent detergent, conditioning, wetting and surface active effects, and which can be widely used in a toiletry or detergent composition or the like.

Alkylaminopropionic acids and their salts are well known in the art as effective foaming agents, detergents, emulsifiers and wetting agents. Compounds of this type are preferred over fatty acid salts because they are tolerant of hard water and high electrolyte levels. Furthermore, unlike fatty acid salts, they remain soluble under acidic conditions and maintain their surface active properties at both low and high pH levels. As disclosed in United States Patent No. 2,195,974 to Reppe, et al.*,* alkylaminopropionic acids of this type may be made by reacting an alkylamine with acrylic acid in aqueous solution. U.S. Patent No. 2,468,012 to Isbell teaches that the alkylaminopropionate salts of alkylaminopropionic acids may be produced by neutralizing with alkali metals, ethanolamines, alkylamines and the like. However, all of these neutralizers suffer from significant drawbacks. Alkali metals such as sodium produce emulsions with poor sheen. Amines such as ethanolamines or alkylamines risk the production of nitrosamine byproducts, which are suspected carcinogens.

Amino acids are not typically used as neutralizers in surfactant salts. However, U.S. Published Patent Application No. 2011/0240050 to Perruna, et al.*,* teaches that arginine may be used to neutralize fatty acids such as coco fatty acid to produce soaps with desirable characteristics without the drawbacks of conventionally neutralized soaps. Yet a significant drawback to fatty acid salts of arginine is that at acidic pH, even slightly acidic solutions typically found in personal care products (*i.e.* pH of 4 to 6), the fatty acid becomes protonated. It is well known that protonated fatty acids are insoluble in water, which leads to a "soap scum" depositing on wash basins and tubs.

Therefore, it is desirable to provide a compound for personal care compositions (such as cosmetics and soaps) that are free from any of the drawbacks of conventional alkylaminopropionates.

### SUMMARY OF THE INVENTION

The present invention provides a compound, an amino acid derived surfactant suitable for use in personal care and other formulations. The surfactant of the present invention is an alkylaminopropionate salt of a basic amino acid represented by formula (I). Such compounds have surprisingly been found to have very low potential to cause irritation while maintaining desirable surfactant properties. 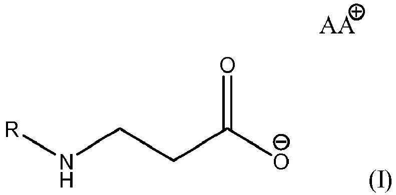 where R represents a linear or branched alkyl or alkenyl group having from eight to 22 carbon atoms and AA+ represents a basic amino acid, e.g. lysine, arginine, or histidine. The basic amino acid may be arginine such that the surfactant is represented by the general formula (II). 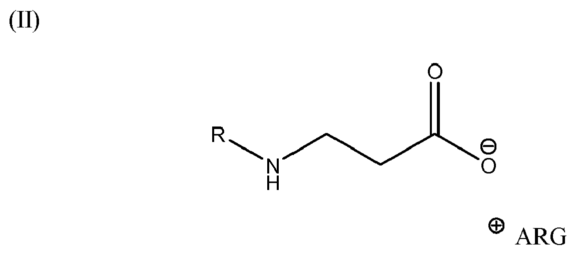 where R represents a linear or branched alkyl or alkenyl group having from 8 to 22 carbon atoms and ARG represents the amino acid Arginine, e.g. L-arginine.

In embodiments R may represent a linear or branched alkyl or alkenyl group having at least 10, at least 12, at least 14, at least 16, at least18 or at least 20 carbon atoms and/or no more than 20, no more than 18, no more than 16, no more than 14, no more than 12, or no more than 10 carbon atoms. In embodiments R may represent a linear alkyl group having from 8 to 22 carbon atoms, such as 10 to 20 carbon atoms, such as 12 to 18 carbon atoms. In one particular embodiment, R represents a linear alkyl group having from 8 to 14 carbon atoms. The alkylaminopropionic acid from which the surfactant is derived may be at least one of lauraminopropionic acid, cocaminopropionic acid, and stearaminopropionic acid.

Lauraminopropionic Acid (CAS Number: 1462-54-0) has Chemical Name: beta.-Alanine, N-dodecyl-, which is EINECS/ELINCS No: 215-968-1. Hence, in the resulting surfactant R is a linear alkyl group having 12 carbon atoms.

Cocaminopropionic Acid (CAS Number: 84812-94-2) has Chemical Name: beta.-Alanine, N-coco alkyl derivs, which is EINECS/ELINCS No: 284-219-9

Stearaminopropionic acid (CAS No. 112-87-8) is also known as beta-Alanine, N-octadecyl- (not on TSCA).

The mole ratio of alkylaminopropionic acid to basic amino acid may be from (0.25 to 2.0) to 1, such as (0.5 to 2.0) to 1, for example 2:1, 1:1 or 2:3.

The invention also resides in a personal care composition comprising the alkylaminopropionic acid salt of a basic amino acid of general formula (I) or (II). The personal care composition may comprise at least 0.01% and/or no more than 50% by weight of the surfactant.

The invention also resides in a composition comprising one of a fire-fighting foam, a hard surface cleaner, a foaming acid cleaner, a basic cleaner, an additive to a textile finish, an antibacterial cleaner, and a foamer for lowering hydrostatic pressure in gas wells, the composition comprising the surfactant (the alkylaminopropionic acid salt of a basic amino acid of formula (I) or (II)).

### BRIEF DESCRIPTION OF THE DRAWINGS

The organization and manner of the structure and operation of the invention, together with further objects and advantages thereof, may best be understood by reference to the following description, taken in connection with the accompanying non-scale drawings, wherein like reference numerals identify like elements in which:
FIG. 1 is a chart of wetting time at two different pH levels for the composition of the preferred embodiment of the present invention and several other common products.
FIG. 2 is a chart of the ZEIN number of the composition of the preferred embodiment of the present invention and several other products.
FIG. 3 is a chart of the level of the foam-water interface for the preferred embodiment of the present invention and several other products.

### DETAILED DESCRIPTION OF THE PREFERRED EMBODIMENTS

It is to be understood that the invention is not limited to the details of construction or process steps set forth in the following description. The invention is capable of other embodiments and of being practiced or being carried out in various ways. Unless indicated otherwise, all amounts of composition ingredients expressed in percentage terms are expressed as weight/weight.

It has been surprisingly discovered that arginine salts of alkylaminopropionic acids exhibit very low potential for skin irritation while demonstrating excellent surfactant properties and are thus ideal candidates for use in personal care and cosmetic formulations. According to the present invention, salts of alkylaminopropionic acids are provided which are represented by the formula (III). 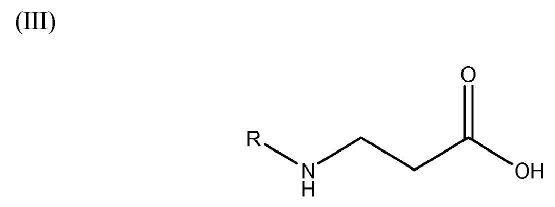 where R represents a linear or branched alkyl or alkenyl group having from 8 to 22 carbon atoms.

Examples of alkylaminopropionic acids include lauraminopropionic acid, cocaminopropionic acid, and stearaminopropionic acid. A preferred embodiment of the present invention uses lauraminopropionic acid.

According to the present invention, compounds containing arginine are provided. Arginine is a semi-essential amino acid. Typically, arginine is found in the L-form (commercially available from Ajinomoto). In an embodiment of the present invention the arginine is a natural or synthetic L-arginine.

When the alkylaminopropionic acid and the arginine are combined, the arginine neutralizes the alkylaminopropionic acid to form a surfactant. Thus, the subjects of the present invention include both an alkylaminopropionic acid and arginine in a combination that provides a salt of the two components.

Zein numbers provide a way of measuring the irritancy of a given compound. Zein numbers are measured by incubating solutions of a compound with solid Zein protein until saturated. Undissolved zein protein was then removed by centrifugation followed by filtration. The amount of zein that has been solubilized can then be measured by standard protein assay methods such as a Pierce BCA protein assay kit. Solubilization of higher amounts of Zein protein results in a higher Zein number, which is associated with greater amounts of irritation. Surprisingly, the arginine salts of the present invention were found to have very low Zein numbers.

Desirable surfactant properties include surface tension reduction, wetting, and foam generation. The compounds of the present invention demonstrate these surface active properties. In particular, it was surprisingly found that the wetting capacity of the inventive compounds was superior to that of other surfactant compounds commonly used in personal care products.

### EXAMPLES

The following examples are intended to further illustrate the present invention. They are not intended to limit the invention in anyway.

### Example 1.

### Preparation of arginine lauraminopropionate

### Preparation A (2:1 mole ratio):

To 300 grams of an aqueous solution containing 100 grams (0.39 moles) of lauraminopropionic acid, were added 56 grams of water and 34 grams (0.195 moles) of arginine. The mixture was mixed at 60 °C until clear and homogeneous. The resulting solution was a pale yellow, clear liquid with a pH of 10.5 and a viscosity at 25 degrees C of 1080 cps.

### Preparation B (1:1 mole ratio)

To 300 grams of an aqueous solution containing 100 grams (0.39 moles) of lauraminopropionic acid, were added 158.4 grams of water and 67.9 grams (0.39 moles) of arginine. The mixture was mixed at 60 °C until clear and homogeneous. The resulting solution was a pale yellow, clear liquid with a pH of 9.9 and a viscosity at 25 degrees C of 1250 cps.

### Preparation C (∼2:3 mole ratio)

To 200 grams of an aqueous solution containing 55 grams (0.21 moles) of lauraminopropionic acid, were added 114 grams of water and 56 grams (0.32 moles) of arginine. The mixture was mixed at 40 °C until clear and homogeneous. The resulting solution was a pale yellow, clear liquid with a pH of 9.6 and a viscosity at 25 °C of 2849 cps.

### Example 2.

### Surface Tension

Surface tension measurements were taken on solutions made from the mixture prepared in Example 1(C) using a du Nouy ring interfacial tensiometer, equipped with a six-cm circumference platinum-iridium ring. A series of test solutions at different concentrations were prepared and their surface tension measured at 25°C. For each test concentration, the ring is slowly drawn out of the liquid. The surface tension is equal to the force required to draw the ring completely out of the liquid, divided by twice the circumference of the ring. The Critical Micelle Concentration (CMC) was found to be 2.46 millimoles per liter. The surface tension at the CMC was 29.6 dyne/cm.

### Example 3.

### Wetting

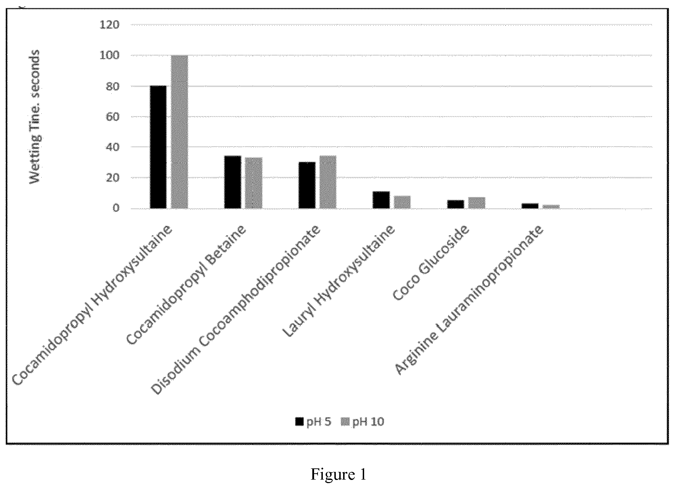

Surfactant wetting was measured according to the Draves cotton skein method on the arginine lauraminopropionate prepared in Example 1 (C) and on several other commercially available surfactants commonly used in personal care products. Cotton skeins weighing 5.0 ± 0.05 grams were attached to a lead weight and completely immersed in solutions containing 0.5% of each test surfactant at 25°C. The time required for the skein to sink was recorded. The results for two different pH levels are shown in FIG. 1.

### Example 4.

### Zein Solubilization:

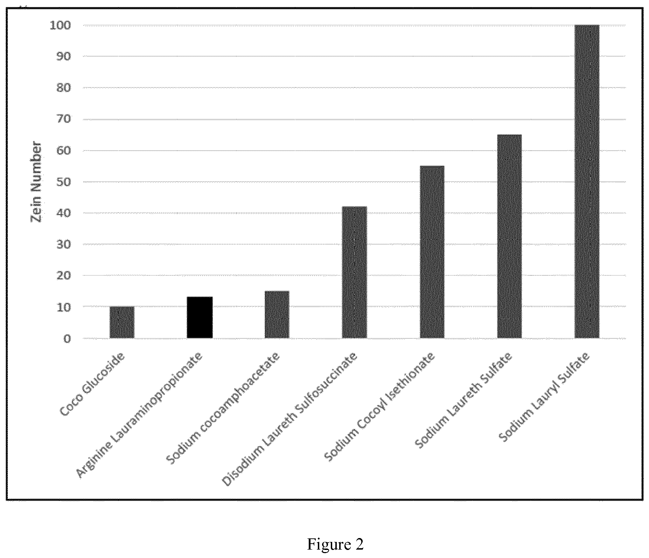

Solutions of the arginine lauraminopropionate prepared in Example 1(C) and on several other commercially available surfactants commonly used in personal care products were prepared at 10 mM in deionized water. To 100 mL of each solution, one gram of zein protein was added and incubated for one hour with mixing at 25°C. Undissolved protein was then removed by centrifugation followed by filtration through a 0.46 µm membrane. The protein concentration of the filtrate was analyzed using a Pierce BCA protein assay kit and a Lange LICO 620 spectral-photometer at wavelength of 560 nm. The results are shown in FIG. 2.

### Example 5.

### Foam Generation

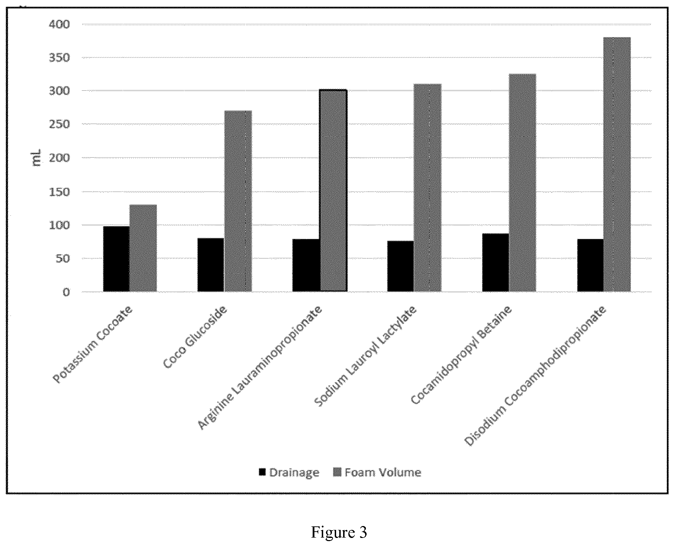

Foam generation and drainage was evaluated on arginine lauraminopropionate prepared in Example 1(C) and on several other commercially available surfactants commonly used in personal care products using a blender foam test. Solutions of test surfactants were prepared at 10 mM in deionized water at 25°C. 100 mL of each solution was placed in a blender (Osterizer) and agitated at high speed for 10 seconds. The foam was poured into a 1000 mL graduated cylinder and the foam volume recorded. After five minutes, the level of the foam-water interface was recorded. The results are shown in FIG 3.

The composition of the present invention can be used in a wide variety of products, such as a hydrotrope and foaming agent for fire-fighting foam, for a hard surface cleaner useful on, for examples, windows, floors, dishwashing, car washing, and for foaming acid and basic cleaners. The composition is also useful as an additive to textile finishes to aid in lubricity and then finish removal (scouring). The composition can be used as a foamer for antibacterial cleaners and as a foamer for lowering hydrostatic pressure in gas wells, particularly in the presence of high and low pH formulations.

The composition of the present invention, due to its amphoteric nature, possesses a good water solubility over a broad pH range (acidic to alkaline) even when high electrolyte concentration is present. Accordingly, the composition of the present invention can be used in a wide variety of products besides personal care products, for examples as a hydrotrope and foaming agent for fire-fighting foam, as a hard surface cleaner useful on, for examples, windows, floors, dishwashing, car washing, and for foaming acid and basic cleaners. The composition is also useful as an additive to textile finishes to aid in lubricity and then finish removal (scouring). The composition can be used as a foamer for antibacterial cleaners and as a foamer for lowering hydrostatic pressure in gas wells, particularly in the presence of high and low pH formulations.

While preferred embodiments of the present invention are shown and described, it is envisioned that those skilled in the art may devise various modifications of the present invention without departing from the scope of the appended claims.

## Claims

1. A alkylaminopropionic acid salt of a basic amino acid of the general formula 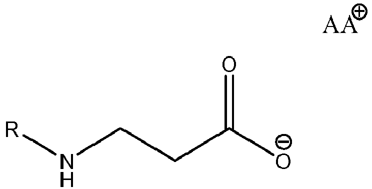 where R represents a linear or branched alkyl or alkenyl group having from eight to 22 carbon atoms and AA+ represents a basic amino acid.

2. The alkylaminopropionic acid salt of a basic amino acid of claim 1 where the basic amino acid is arginine.

3. The alkylaminopropionic acid salt of a basic amino acid of claim 1 or 2 where the basic amino acid is L-arginine.

4. The alkylaminopropionic acid salt of a basic amino acid of any of claims 1, 2, or 3 where the alkylaminopropionic acid is at least one of lauraminopropionic acid, cocaminopropionic acid, and stearaminopropionic acid.

5. The alkylaminopropionic acid salt of any one of the preceding claims where R represents a linear alkyl group having from 8 to 22 carbon atoms.

6. The alkyl aminopropionic acid salt of claim 5, where R represents a linear alkyl group having from 12 to 18 carbon atoms.

7. The alkylaminopropionic acid salt of a basic amino acid of any of the preceding claims where the mole ratio of alkylaminopropionic acid to basic amino acid is (0.5 to 2.0) to 1.

8. A personal care composition comprising the alkylaminopropionic acid salt of a basic amino acid of any of the preceding claims.

9. The personal care composition of claim 8 comprising about between 0.01 and 50% by weight of the alkylaminopropionic acid salt of a basic amino acid of any one of claims 1 to 7.

10. A composition comprising one of a fire-fighting foam, a hard surface cleaner, a foaming acid cleaner, a basic cleaner, an additive to a textile finish, an antibacterial cleaner, and a foamer for lowering hydrostatic pressure in gas wells, the composition comprising the alkylaminopropionic acid salt of a basic amino acid of any one of claims 1 to 7.
